# EUROPÄISCHE PATENTSCHRIFT

(11) **EP 1 971 276 B1**
(45) Veröffentlichungstag und Bekanntmachung des Hinweises auf die Patenterteilung: **20.04.2011**
(21) Anmeldenummer: 06762529.3
(22) Anmeldetag: 11.07.2006
(51) Int. Cl.: A61B 17/122

(54) **CHIRURGISCHER LIGATURCLIP**
SURGICAL LIGATURE CLIP
PINCE DE LIGATURE CHIRURGICALE

(30) Priorität: 11.01.2006 DE 202006000329 U
(43) Veröffentlichungstag der Anmeldung: 24.09.2008
(60) Teilanmeldung: 08163808.2
(73) Patentinhaber: Aesculap AG, 78532 Tuttlingen (DE)
(72) Erfinder: LUTZE, Theodor, 78582 Balgheim (DE); DWORSCHAK, Manfred, 78589 Dürbheim (DE)
(74) Vertreter: Hoeger, Stellrecht & Partner Patentanwälte
(86) Internationale Anmeldenummer: PCT/EP2006/006781
(87) Internationale Veröffentlichungsnummer: WO 2007/087834

(56) Entgegenhaltungen:
- EP-A- 0 567 965
- WO-A-98/18389
- DE-A- 2 405 390
- US-A- 5 171 250
- US-A- 5 788 716
- "Titan Ligatur Clips und Anlegezangen" [Online] Februar 2002 (2002-02), AESCULAP , TUTTLINGEN DEUTSCHLAND , XP002400693 Gefunden im Internet: URL:http://www.mediwar.ch/d/produkte/chiru rgie/documents/TitanLigaturClips.pdf#searc h=%22titanligaturclips%20aesculap%22> [gefunden am 2006-09-26] in der Anmeldung erwähnt Prospektblatt C46811 Seiten 6-7

## Beschreibung

Die Erfindung betrifft einen chirurgischen Ligaturclip mit zwei Haltearmen, die an jeweils einem Ende über eine verformbare Verbindungsstelle miteinander verbunden sind und derart gegeneinander biegbar sind, daß die Arme aus einer Offenstellung, in der sie einen größeren Abstand voneinander haben, in eine Schließstellung gelangen, in der die einander zugewandten Innenseiten der Arme dauerhaft einander angenähert sind, wobei der Ligaturclip aus einem in sich geschlossenen Steg besteht, der im Bereich der beiden Arme und der Verbindungsstelle zwei nebeneinander liegende Abschnitte ausbildet, die an den der Verbindungsstelle abgewandten freien Enden der Arme ineinander übergehen.

Sowohl in der offenen als auch in der minimal-invasiven Chirurgie zählt die Applikation von Ligaturclips zum sicheren und schnellen Verschluß von Blutgefäßen und anderen Hohlorganen, wie z.B. von Gallengängen, zu einem bewährten Verfahren. Die Ligatur von Gefäßen und Hohlorganen ist dann erforderlich, wenn Gewebeanteile reseziert werden sollen und wenn bei der Durchtrennung von Gewebe eine Blutung entsteht, die mittels Koagulation nur schwer oder unsicher beherrscht werden kann.

Dabei kommen überwiegend Clips aus Reintitan zum Einsatz. Dies ist ein erprobter Werkstoff mit ausgezeichneten Biokompatibilitätseigenschaften. Es sind aber auch Clips aus resorbierbaren Materialien bekannt, dabei handelt es sich meist um Clips aus Polylactaten oder Clips aus anderen Kunststoffen, beispielsweise Polyetheretherketon.

Ligaturclips dieser Art werden beispielsweise vertrieben von der Firma AESCULAP AG & Co. KG unter dem Markennamen Challenger Ti (Prospektblätter der Firma AESCULAP AG & Co. KG "Titanligaturclips und Anlegezangen" C 468 11, Veröffentlichungsdatum September 2003; Challenger Ti, C461 11, Veröffentlichungsdatum Februar 2002).

Bekannte Clips dieser Art sind häufig V-förmig ausgebildet, die Breite der Clips variiert je nach Größe zwischen 0,5 und 1,2 Millimetern. Die Innenflächen sind in der Regel mit einer Profilierung versehen, um einen besseren Sitz auf dem Hohlorgan in Längsrichtung zu bewirken.

Trotz dieser Ausbildung besteht bei bekannten Clips die Gefahr, daß sie von dem geclipten Hohlorgan abgleiten. Üblicherweise werden die Hohlorgane nach dem Anlegen eines Ligaturclips dicht neben dem Clip durchtrennt, und dann kann es passieren, daß der Clip unbeabsichtigt seitlich von dem Hohlorgan heruntergeschoben wird, beispielsweise bei der Entnahme von Bauchtüchern, die über geclipten Gefäßen positioniert werden oder bei der minimal-invasiven Chirurgie durch Instrumente, die sich außerhalb des Sichtbereichs des Operateurs bewegen und daher von ihm nur schlecht kontrolliert werden können.

Aus der US-A-5171250, der WO-A-9818389 und der EP-A-0567965 sind Ligaturclips bekannt, bei denen die beiden Arme zwei nebeneinander liegende Abschnitte ausbilden, die an den der Verbindungsstelle abgewandten freien Enden der Arme ineinander übergehen.

Man erhält auf diese Weise einen Doppelclip mit zwei nebeneinander liegenden Armteilen, durch diese doppelte Ausbildung der Armteile wird die Anlage des Ligaturclips am Hohlorgan deutlich verbessert. Man könnte einen solchen Ligaturclip auch betrachten als eine Baueinheit aus zwei nebeneinander liegenden Ligaturclips, die im Bereich des freien Endes der Arme durch eine Brücke miteinander verbunden sind. Die beiden Teile des Clips stabilisieren sich dadurch gegenseitig, so daß die Halteeigenschaften des am Hohlorgan angelegten Ligaturclips so gut sind, daß im allgemeinen darauf verzichtet werden kann, entsprechend der bisherigen Praxis an einem im Körper verbleibenden Hohlorgan unabhängig voneinander nebeneinander zwei derartige Ligaturclips zu plazieren.

Ausgehend von diesem Stand der Technik ist es Aufgabe der Erfindung, einen gattungsgemäßen Ligaturclips so auszubilden, dass sein Sitz auf dem geclipten Hohlorgan weiter verbessert wird.

Diese Aufgabe wird bei einem Ligaturclip der eingangs beschriebenen Art erfindungsgemäß dadurch gelöst, daß die nebeneinander liegenden Abschnitte an ihrem der Verbindungsstelle abgewandten Ende in nach gegenüberliegenden Seiten seitlich abstehende Transversalabschnitte einmünden. Es ergibt sich dadurch eine insgesamt T-förmige Ausgestaltung der Arme, wobei die Transversalabschnitte beispielsweise Klemmbacken ausbilden können.

Ein derartiger Ligaturclip ist beispielsweise besonders geeignet zur Verbindung von zwei Hohlorganen, die an den aneinander stoßenden Enden mit ihrem Rand seitlich nach außen umgebogen werden, so dass ein flanschförmiger Überstand entsteht. In diesem Bereich können mehrere Ligaturclips in Umfangrichtung nebeneinander derart angeordnet werden, daß die seitlich abstehenden Transversalabschnitte der Außenkontur der Hohlorgane folgen.

Es ist günstig, wenn der Steg über die gesamte Länge eine gleichbleibende Breite und/oder Höhe aufweist. Insbesondere kann der Steg über seine gesamte Länge einen gleichbleibenden Querschnitt aufweisen. Dieser kann beispielsweise rechteckig sein.

Bei einer bevorzugten Ausführungsform ist vorgesehen, daß die nebeneinander liegenden Abschnitte geradlinig ausgebildet sind und insbesondere parallel zueinander verlaufen.

Günstig ist es weiterhin, wenn die Transversalabschnitte eines Armes an ihren den nebeneinander liegenden Abschnitten abgewandten äußeren Enden in einen Klemmabschnitt übergehen, der im Abstand von den Transversalabschnitten auf deren den nebeneinander liegenden Abschnitten abgewandter Seite verläuft. Die Transversalabschnitte und der diese verbindende Klemmabschnitt bilden somit eine Klemmfläche am freien Ende der Arme aus, die nach beiden Seiten von den Armen absteht. Zwischen dem Klemmabschnitt einerseits und den Transversalabschnitten andererseits besteht ein Zwischenraum ähnlich wie zwischen den beiden nebeneinander liegenden Abschnitten der Arme, so daß in diesem Bereich geklemmtes Gewebe in den Zwischenraum eindringen kann. Dies führt zu einer besonders sicheren Anlage der aus Klemmabschnitt und den beiden Transversalabschnitten ausgebildeten Klemmfläche.

Insbesondere kann vorgesehen sein, daß der Klemmabschnitt im wesentlichen parallel zu den Transversalabschnitten verläuft.

Die Transversalabschnitte können an den äußeren Enden bogenförmig in den Klemmabschnitt übergehen, insbesondere kreisbogenförmig. Es ergibt sich dadurch eine atraumatische Ausgestaltung der Klemmflächen im Bereich der äußeren Enden.

Vorzugsweise liegen die Transversalabschnitte und gegebenenfalls der Klemmabschnitt in derselben Ebene wie die an die Transversalabschnitte anschließenden nebeneinander liegende Abschnitte.

Der Klemmabschnitt kann geradlinig ausgebildet sein, gemäß einer besonders bevorzugten Ausführungsform verläuft er jedoch bogenförmig, insbesondere kreisbogenförmig.

Dabei ergibt sich eine besonders günstige Ausgestaltung, wenn der Klemmabschnitt von den nebeneinander liegenden Abschnitten wegweisend gebogen ist, wenn also die Mitte des Klemmabschnittes näher an den nebeneinander liegenden Abschnitten liegt als die äußeren Enden. Ein derartiger Clip kann besonders günstig an die Außenseite eines Hohlorganes angelegt werden, da der bogenförmige Verlauf des Klemmabschnittes dem gebogenen Außenverlauf des Hohlorgans folgen kann.

Vorzugsweise werden die Ligaturclips mit Klemmabschnitten unterschiedlicher Krümmung gefertigt, das heißt es gibt in einem Satz mehrere Ligaturclips mit unterschiedlicher Krümmung, so daß die Clips an die jeweiligen Abmessungen der Hohlorgane angepaßt eingesetzt werden können.

Gemäß einer bevorzugten Ausführungsform ist vorgesehen, daß mindestens ein Klemmabschnitt einen Vorsprung trägt, der bei aneinander liegenden Klemmabschnitten der beiden Arme in eine Ausnehmung des anderen Klemmabschnitt eintaucht. Dadurch werden einerseits die Klemmabschnitte beim Annähern relativ zueinander geführt, zum anderen durchdringen die Vorsprünge, die insbesondere als Stift ausgebildet sein können, das zwischen den Klemmabschnitten gehaltene Gewebe und fixieren dadurch Gewebe und Ligaturclip relativ zueinander.

An dem Klemmabschnitt eines Armes können über dessen Längsrichtung verteilt mehrere Vorsprünge und an dem Klemmabschnitt des anderen Armes entsprechende Ausnehmungen angeordnet sein, so daß eine Fixierung des Gewebes über die gesamte Länge des Klemmabschnittes erfolgt.

Es ist günstig, wenn der Steg an seiner die Innenseite der Arme ausbildenden Seite profiliert ist. Dadurch ergibt sich ein sicherer Sitz auf einem Hohlorgan.

Insbesondere kann die Profilierung im Bereich des freien Endes der Arme eine Mauszahnprofilierung sein, also ineinander greifende Zähne aufweisen . Dadurch wird sicher verhindert, daß das zwischen den Armen gefaßte Hohlorgan aus dem Zwischenraum zwischen den Armen herausgleiten kann.

Bei einer weiteren bevorzugten Ausführungsform ist vorgesehen, daß die Arme an ihrem freien Ende eine Rastvorrichtung tragen, die beim Annähern der Arme in diesem Bereich zu einer Rastverbindung der Arme führt. Auch dies stellt sicher, daß das gefaßte Hohlorgan nicht seitlich aus dem Zwischenraum zwischen den Armen herausgleiten kann.

Günstig ist es, wenn die Rastvorrichtung mindestens einen Vorsprung an einem Arm umfaßt, der bei geschlossenem Ligaturclip einen Teil des anderen Armes hintergreift.

Beispielsweise kann der Vorsprung an seinem freien Ende eine Verbreiterung oder eine Abwinkelung aufweisen oder als Haken ausgebildet sein.

Es ist besonders vorteilhaft, wenn der Steg ein einteiliges Bauteil ist. Beispielsweise kann es sich bei dem Steg um ein in sich geschlossenes Band aus Titan oder einer Titanlegierung handeln, dieses in sich geschlossene Band wird durch Faltung um eine Mittellinie gebogen, im Bereich der Mittellinie bildet dann der Steg die Verbindungsstelle des Ligaturclips aus. Dabei kann man ausgehen von einem kreisringförmigen Band und erhält dann einen relativ breiten Ligaturclip mit kreisbogenförmig gebogenen Abschnitten der Arme, oder auch von einem Band mit parallelen, geradlinigen Abschnitten, die an ihren Enden bogenförmig miteinander verbunden sind. Im letzteren Fall erhält man einen Clip mit parallelen, geradlinigen Abschnitten des Steges in den Armen mit einem Zwischenraum zwischen den Stegen in Form eines parallelen Längsschlitzes.

Bei einer bevorzugten Ausführungsform der Erfindung kann vorgesehen sein, daß die beiden nebeneinander liegenden Abschnitte im Bereich der Verbindungsstelle durch einen Steg miteinander verbunden sind. Dadurch wird die Stabilität des Ligaturclips erhöht, insbesondere wird verhindert, daß die beiden nebeneinander liegenden Abschnitte bei der Verformung des Ligaturclips ihren Abstand verändern.

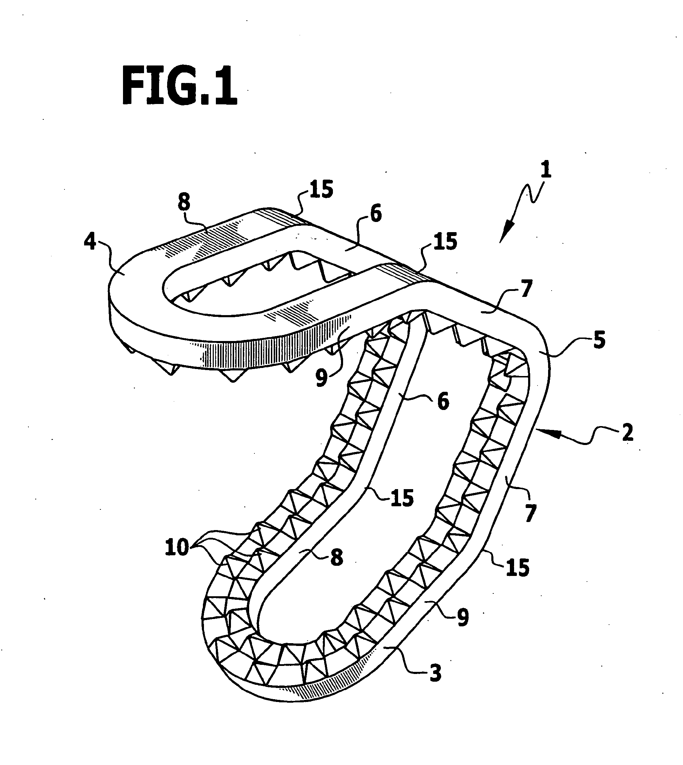

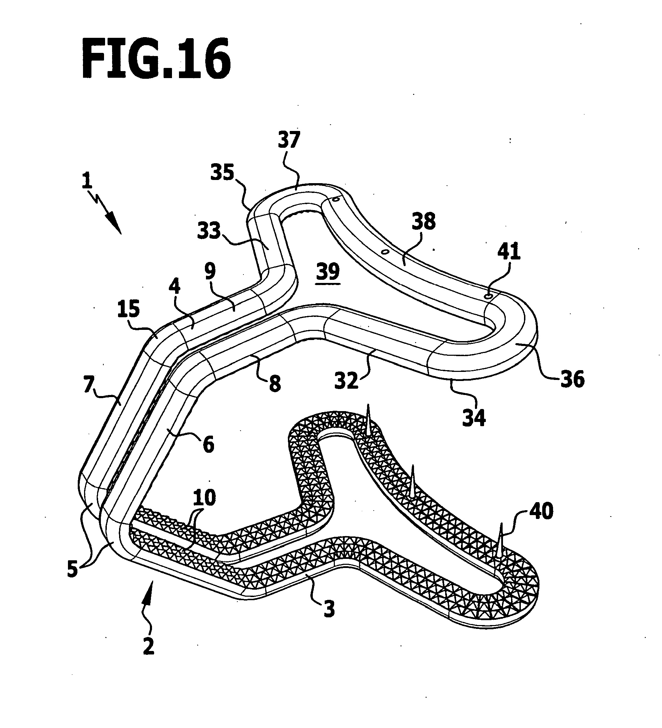

Die nachfolgende Beschreibung bevorzugter Ausführungsformen der Erfindung dient im Zusammenhang mit der Zeichnung der näheren Erläuterung. Dabei zeigen lediglich die Figuren 16 bis 18 Ligaturclips gemäß der Erfindung, die Figuren 1 bis 15 dienen nur der Erläuterung des Aufbaus der Ligaturclips wie sie in den Figuren 16 bis 18 dargestellt sind.
- Figur 1:: einen Ligaturclip mit zwei nebeneinander liegenden Steg- abschnitten in Offenstellung;
- Figur 2:: eine Teilansicht eines Anlegeinstrumentes mit einem Liga- turclip gemäß Figur 1 in Offenstellung des Ligaturclips;
- Figur 3:: eine perspektivische Teilansicht des Klemmbackens eines abgewandelten Ausführungsbeispiels eines Anlegeinstru- mentes ohne Ligaturclip;
- Figur 4:: eine Ansicht ähnlich Figur 3 mit zwischen beiden Klemm- backen eingelegtem Ligaturclip vor dem Schließen des Li- gaturclips;
- Figur 5:: einen Ligaturclip gemäß Figur 1 in der Anlage an einem Hohlorgan und in Schließstellung;
- Figur 6:: eine Ansicht ähnlich Figur 1 bei einem abgewandelten Aus- führungsbeispiel eines Ligaturclips mit einer Mauszahnprofi- lierung an den freien Enden der beiden Arme;
- Figur 7: eine Ansicht ähnlich Figur 5 bei einem abgewandelten Aus- führungsbeispiel mit einer weiteren Mauszahnprofilierung;
- Figur 8:: eine Ansicht eines Ligaturclips ähnlich Figur 1 mit einem Rastvorsprung an dem freien Ende eines Armes;
- Figur 9: eine Teilansicht eines abgewandelten Ausführungsbeispiels eines derartigen Rastvorsprunges vor dem Eintauchen in eine Rastausnehmung des anderen Armes des Ligaturclips;
- Figur 10:: eine Ansicht ähnlich Figur 7 mit einem hakenförmigen Rast- element an einem Arm des Ligaturclips;
- Figur 11a und Figur 11b:: eine Draufsicht bzw. eine Seitenansicht eines bandförmigen Materials vor dem Biegen zu einem Ligaturclip;
- Figur 12:: eine Seitenansicht des Ligaturclips der Figur 9 in geöffne- tem Zustand;
- Figur 13:: eine Seitenansicht des Ligaturclips der Figur 12 in teilweise geschlossenem Zustand;
- Figur 14:: eine Seitenansicht des Ligaturclips der Figur 12 in ge- schlossenem Zustand;
- Figur 15:: eine perspektivische Ansicht eines Trägers mit einer größe- ren Anzahl von Ligaturclips gemäß Figur 1;
- Figur 16:: eine perspektivische Ansicht eines erfindungsgemäßen Aus- führungsbeispiels eines Ligaturclips mit T-förmiger Ausbil- dung der Arme in geöffnetem Zustand;
- Figur 17:: eine schematische Ansicht von zwei Hohlorganen, die mit- tels Ligaturclips gemäß Figur 16 miteinander verbunden sind und
- Figur 18:: eine Schnittansicht durch den Anlagebereich von zwei Hohlorganen längs Linie 18-18 in Figur 17.
Der in den Figuren 1 bis 5 dargestellte Ligaturclip 1 besteht aus einem in sich geschlossenen streifenförmigen Band 2 aus einem dauerhaft biegbaren Material, beispielsweise aus Titan, einer Titanlegierung oder einem der zur Herstellung von derartigen Ligaturclips üblicherweise verwendeten Kunststoffe. Selbstverständlich können auch andere Materialien Verwendung finden, beispielsweise Stähle, sofern die verwendeten Materialien körperkompatibel sind.

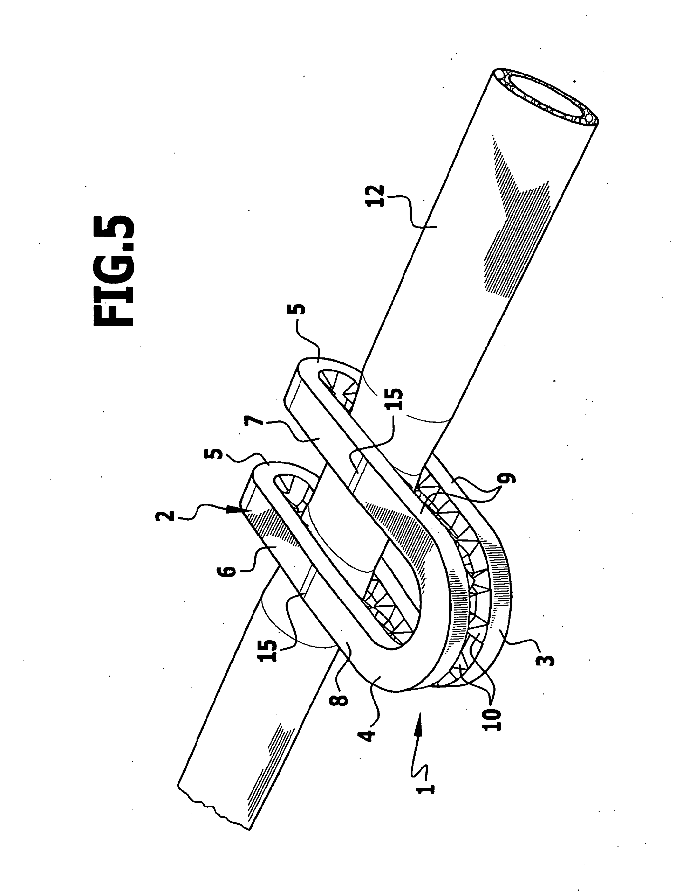

- Figur 5:: einen Ligaturclip gemäß Figur 1 in der Anlage an einem Hohlorgan und in Schließstellung;

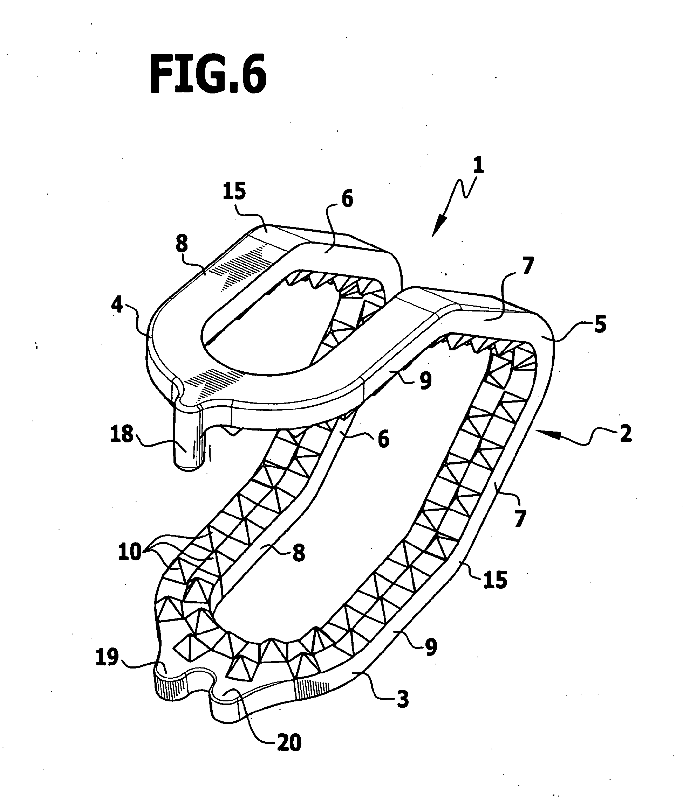

- Figur 6:: eine Ansicht ähnlich Figur 1 bei einem abgewandelten Aus- führungsbeispiel eines Ligaturclips mit einer Mäuszahnprofi- lierung an den freien Enden der beiden Arme;

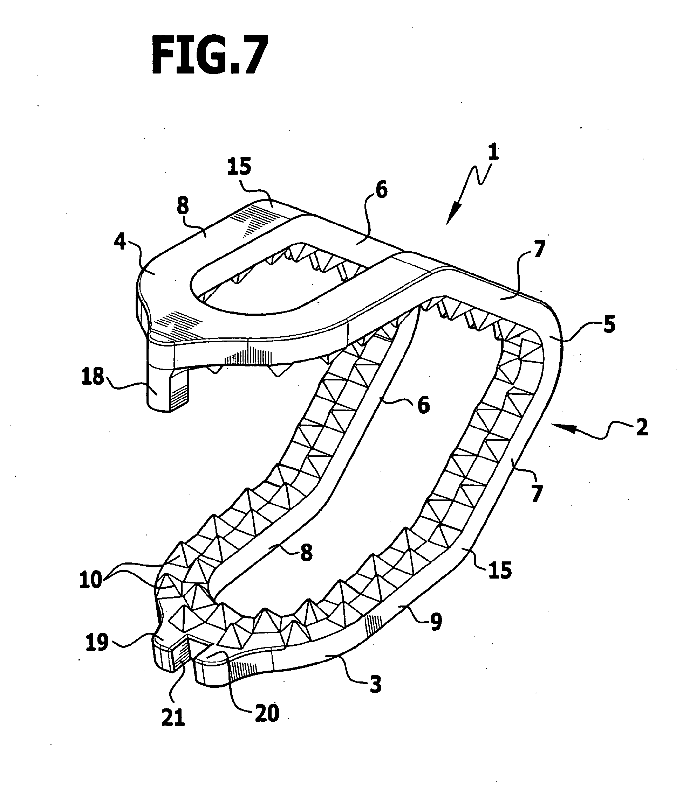

- Figur 7: eine Ansicht ähnlich Figur 5 bei einem abgewandelten Aus- führungsbeispiel mit einer weiteren Mauszahnprofilierung;

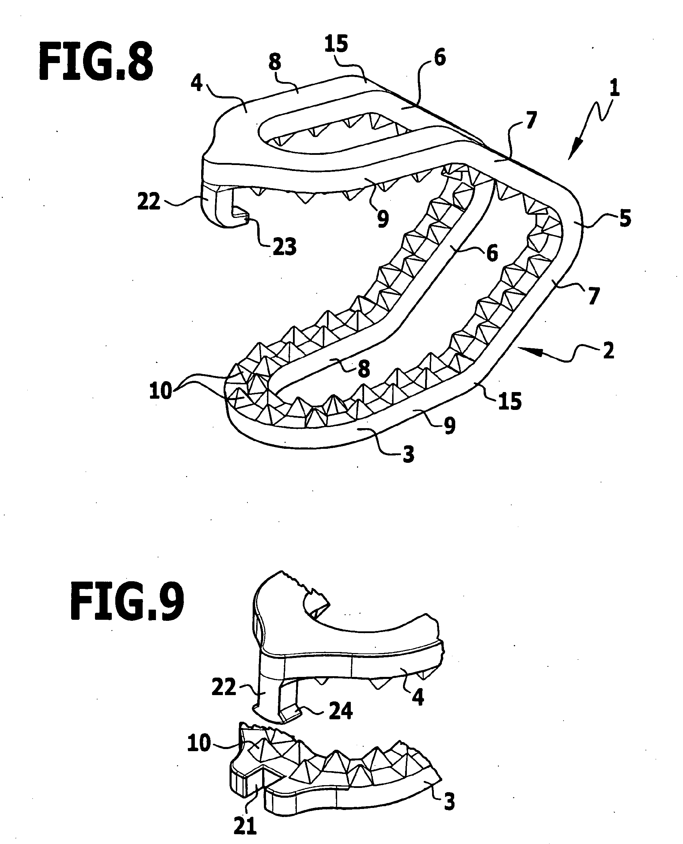

- Figur 8:: eine Ansicht eines Ligaturclips ähnlich Figur 1 mit einem Rastvorsprung an dem freien Ende eines Armes;
- Figur 9: eine Teilansicht eines abgewandelten Ausführungsbeispiels eines derartigen Rastvorsprunges vor dem Eintauchen in eine Rastausnehmung des anderen Armes des Ligaturclips;

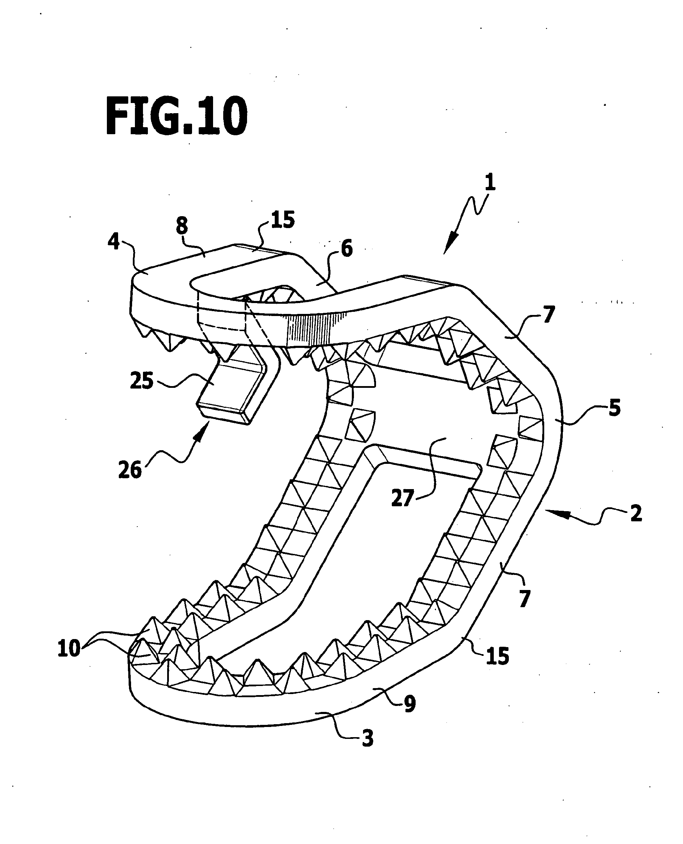

- Figur 10:: eine Ansicht ähnlich Figur 7 mit einem hakenförmigen Rast- element an einem Arm des Ligaturclips;

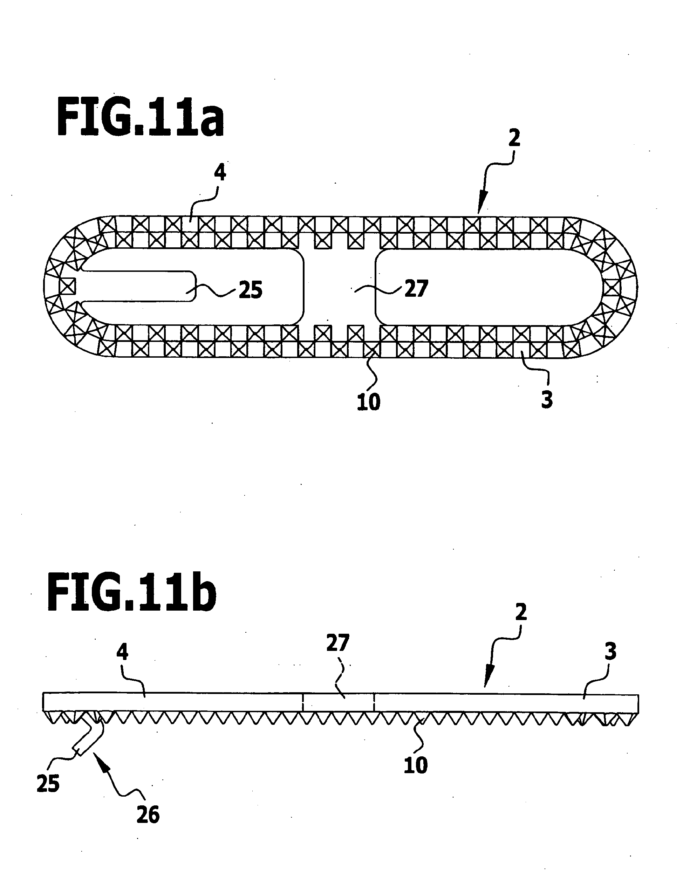

- Figur 11a und Figur 11b:: eine Draufsicht bzw. eine Seitenansicht eines bandförmigen Materials vor dem Biegen zu einem Ligaturclip;

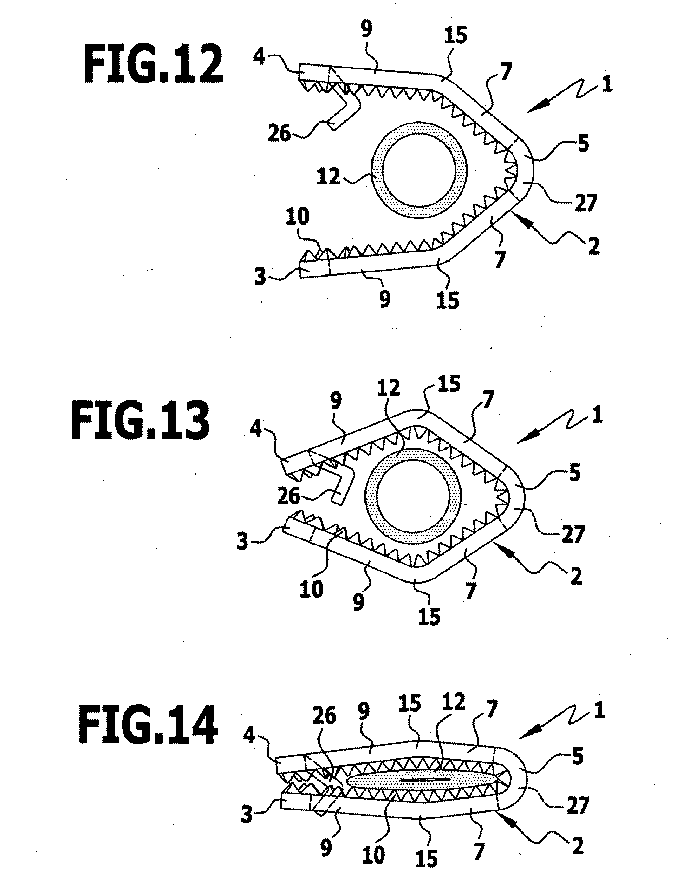

- Figur 12:: eine Seitenansicht des Ligaturclips der Figur 9 in geöffne- tem Zustand;
- Figur 13:: eine Seitenansicht des Ligaturclips der Figur 12 in teilweise geschlossenem Zustand;
- Figur 14:: eine Seitenansicht des Ligaturclips der Figur 12 in geschlos- senem Zustand;

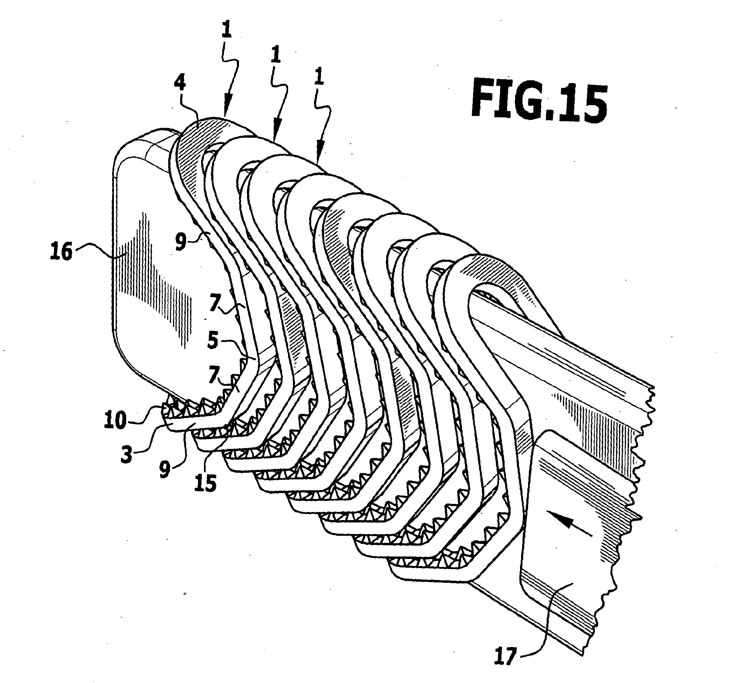

- Figur 15:: eine perspektivische Ansicht eines Trägers mit einer größe- ren Anzahl von Ligaturclips gemäß Figur 1;
- Figur 16:: eine perspektivische Ansicht eines weiteren bevorzugten Ausführungsbeispiels eines Ligatürclips mit T-förmiger Ausbildung der Arme in geöffnetem Zustand;

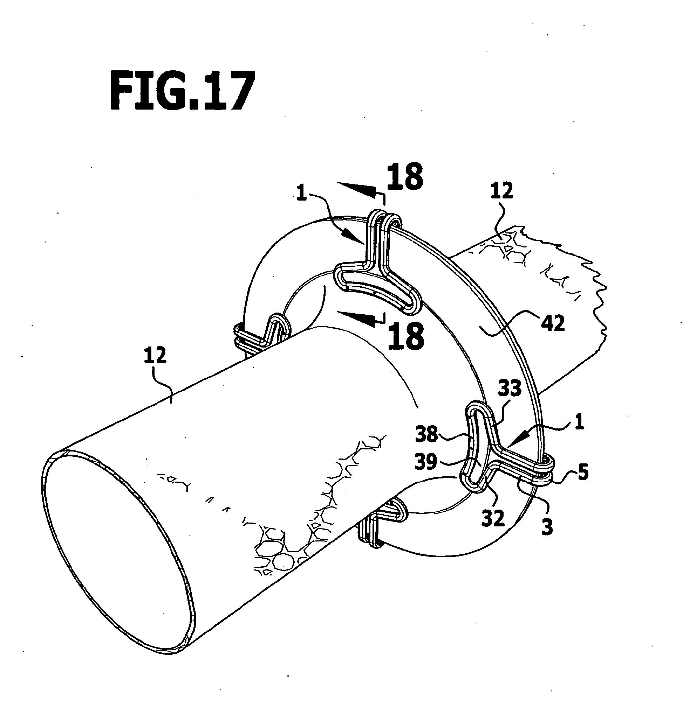

- Figur 17:: eine schematische Ansicht von zwei Hohlorganen, die mit- tels Ligaturclips gemäß Figur 16 miteinander verbunden sind und

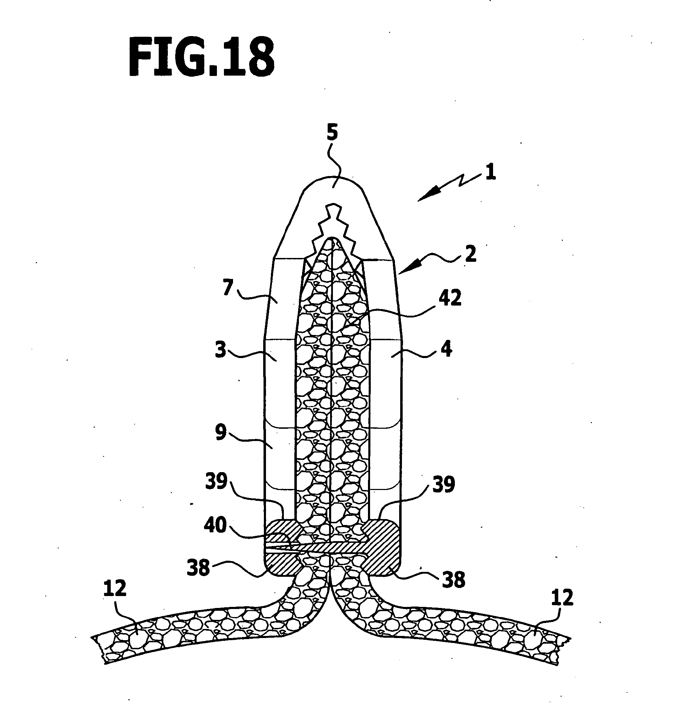

- Figur 18:: eine Schnittansicht durch den Anlagebereich von zwei Hohlorganen längs Linie 18-18 in Figur 17.
Der in den Figuren 1 bis 5 dargestellte Ligaturclip 1 besteht aus einem in sich geschlossenen streifenförmigen Band 2 aus einem dauerhaft biegbaren Material, beispielsweise aus Titan, einer Titanlegierung oder einem der zur Herstellung von derartigen Ligaturclips üblicherweise verwendeten Kunststoffe. Selbstverständlich können auch andere Materialien Verwendung finden, beispielsweise Stähle, sofern die verwendeten Materialien körperkompatibel sind. Im dargestellten Ausführungsbeispiel hat das Band 2 einen rechteckigen Querschnitt, der über die gesamte Länge des Bandes 2 gleich bleibt und bei dem die Breite des Bandes etwa doppelt so groß ist wie die Höhe des Bandes.

Das in sich geschlossene Band 2 ist dabei so angeordnet, daß es über einen großen Teil seiner Länge zwei geradlinige, parallel im Abstand zueinander nebeneinander laufende Bereiche ausbildet, die an ihren Enden durch einen kreisbogenförmigen Verlauf des Bandes ineinander übergehen. Längs einer quer zur Längsausdehnung der geradlinigen Bereiche verlaufenden Mittellinie sind die beiden Hälften des in sich geschlossenen Bandes 2 aufeinander gefaltet, so daß sich zwei einander gegenüberstehende Arme 3, 4 ergeben, die im Bereich der genannten Mittellinie eine im wesentlichen bogenförmig verformte Verbindungsstelle 5 ausbilden. Ausgehend von dieser Verbindungsstelle 5 verlaufen die beiden parallel nebeneinander liegenden Teile des Bandes 2 in Form von geradlinigen Abschnitten 6, 7 in einer Ebene, daran schließen sich weitere geradlinige Abschnitte 8, 9 an, die ebenfalls in einer Ebene liegen, wobei die beiden Ebenen etwa um einen Winkel von 30° gegeneinander geneigt sind. Die beiden Abschnitte 8, 9 gehen im Bereich des freien Endes der Arme 3, 4 in Form eines sich über 180° erstreckenden Kreisbogens ineinander über.

Auf der Innenseite, die jeweils dem anderen Arm zugewandt ist, tragen beide Arme 3, 4 eine Profilierung in Form von in Richtung des anderen Armes abstehenden, spitz zulaufenden Zähnen 10, diese Profilierung erstreckt sich über die gesamte Länge des Bandes 2.

In Figur 6 ist ein Ligaturclip 1 dargestellt, der im wesentlichen dem der Figur 1 entspricht, einander entsprechende Teile tragen daher dieselben Bezugszeichen. Im Unterschied zu dem Ligaturclip der Figur 1 tragen die beiden Arme 3, 4 des Ligaturclips 1 an ihrem freien Ende zusätzliche Vorsprünge 18, 19, 20, die so angeordnet sind, daß bei geschlossenem Ligaturclip der Vorsprung 18 an einem der beiden Arme zwischen die Vorsprünge 19, 20 am anderen Arm eintaucht und dadurch einerseits den Zwischenraum zwischen den beiden Armen 3, 4 nach vorne hin abschließt und andererseits die beiden Arme gegen seitliche Verschiebung relativ zueinander sichert.

Bei dem Ligaturclip der Figur 7 ist eine sehr ähnliche Ausgestaltung gewählt, dabei sind die Vorsprünge 18, 19, 20 noch prägnanter ausgebildet, so daß der Vorsprung 18 an einem Arm in eine Ausnehmung 21 zwischen den Vorsprüngen 19, 20 am anderen Arm formschlüssig eintaucht. Eine solche Passung wird auch als Mauszahnprofilierung bezeichnet.

In Figur 8 ist ein ähnlicher Ligaturclip 1 gezeigt, bei dem die beiden Arme im Schließzustand miteinander verrastet werden können. Dazu trägt einer der beiden Arme an seinem freien Ende einen Rastvorsprung 22, der an seinem freien Ende seitlich abgewinkelt ist. Im geschlossenen Zustand kann die seitliche Abwinkelung 23 des Rastvorsprunges 22 am anderen Arm vorbeigleiten und diesen hintergreifen.

In Figur 9 ist eine abgewandelte Ausgestaltung einer Rastvorrichtung dargestellt, dort ist der Rastvorsprung 22 an seinem freien Ende seitlich verdickt. Der Rastvorsprung 22 greift bei geschlossenem Ligaturclip in eine Ausnehmung 21 des anderen Armes ein, und die Verdickung 24 am freien Ende des Rastvorsprungs 22 hintergreift den anderen Arm. Diese Rasteinrichtung übernimmt gleichzeitig eine ähnliche Funktion wie eine Mauszahnprofilierung, die beiden Arme werden nämlich auch noch gegen eine seitliche Verschiebung gegeneinander gesichert.

Bei dem in Figur 10 dargestellten Ligaturclip trägt einer der beiden Arme eine vom freien Ende in den Zwischenraum zwischen den nebeneinanderliegenden Abschnitten ragende Zunge 25, die etwa in der Mitte ihrer Länge um fast 180° umgebogen ist und somit einen Rasthaken 26 ausbildet, der bei geschlossenem Ligaturclip in den Zwischenraum zwischen den nebeneinanderliegenden Abschnitten des anderen Armes eintaucht und diesen Arm hintergreift. Auf diese Weise werden ebenfalls beide Arme im Schließzustand miteinander verrastet.

Zur Herstellung des Ligaturclips 1 wird ausgegangen von einem in sich geschlossenen Band, das in einer Ebene liegt und das mit geradlinigen Abschnitten und kreisbogenförmigen Verbindungsbereichen vorgeformt ist. In den Figuren 11a, 11b sowie 12 bis 14 wird dieser Herstellungsvorgang am Beispiel des Ligaturclips der Figur 10 dargestellt. Das Ausgangsbauteil in Form eines geschlossenen Bandes wird einmal um die Mittellinie und zum anderen im Bereich zwischen den Abschnitten 6 und 8 bzw. 7 und 9 so gebogen, daß die beiden durch diese Abschnitte jeweils aufgespannten Ebenen gegeneinander geneigt sind. Die sich unmittelbar an die Verbindungsstelle 5 anschließenden Abschnitte 6 bzw. 7 der beiden Arme 3, 4 bilden einen Öffnungswinkel von etwa 60° aus, diese Stellung wird als Offenstellung des Ligaturclips 1 bezeichnet. In dieser Stellung der Arme 3 ist der Ligaturclip 1 im Querschnitt etwa V-förmig ausgebildet und ist geeignet, mit Hilfe eines Anlegeinstrumentes 11 seitlich an ein Hohlorgan 12 angelegt zu werden, an dem er festgelegt werden soll.

Der Ligaturclip der Figuren 10, 11a und 11b weist im Bereich der Verbindungsstelle 5 einen Quersteg 27 auf, durch den die Stabilität des Ligaturclips erhöht wird, unbedingt notwendig ist dieser Quersteg allerdings nicht, die Ausführungsbeispiele der Figuren 1, 6, 7 und 8 haben keinen derartigen Quersteg.

Die Figuren 12, 13 und 14 zeigen die Form des Ligaturclips beim Anlegen an ein Hohlorgan 12 während des Schließvorganges bzw. am Ende des Schließvorganges. Man erkennt deutlich, daß bei geschlossenem Ligaturclip der Rasthaken 26 den gegenüberliegenden Arm an dessen freiem Ende hintergreift und somit den Ligaturclip im Bereich der freien Enden der Arme verschließt und außerdem die beiden Arme gegen eine seitliche Verschiebung sichert.

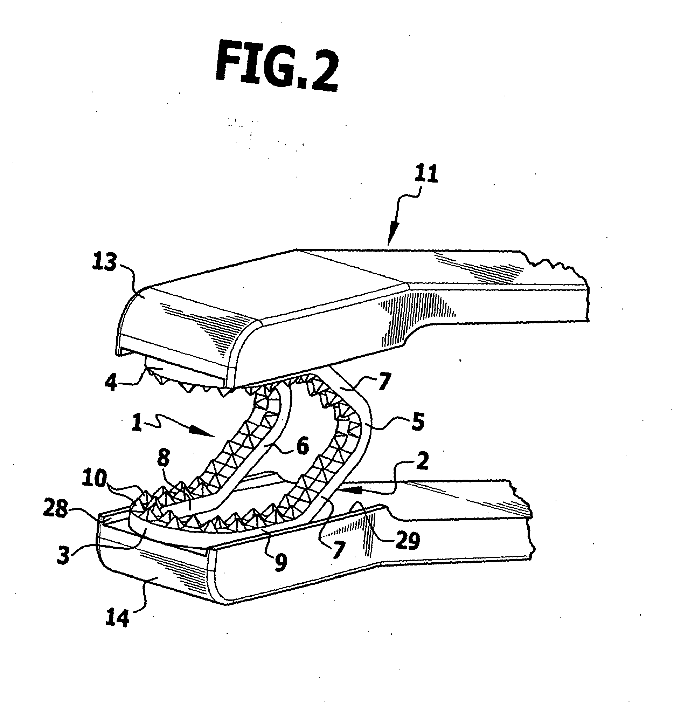

In Figur 2 ist schematisch ein Anlegeinstrument 11 mit einem eingelegten Ligaturclip 1 in Offenstellung dargestellt. Das Anlegeinstrument 11 umfaßt dabei zwei an die Außenseite der beiden Arme 3, 4 anlegbare Klemmbacken 13, 14, die mittels eines in der Zeichnung nicht dargestellten Mechanismus einander angenähert werden können, so daß dadurch die beiden auf gegenüberliegenden Seiten eines Hohlorgans 12 angeordneten Arme 3, 4 gegeneinandergedrückt werden. Dabei wird der Ligaturclip 1 im Bereich der Verbindungsstelle 5 und gegebenenfalls auch im Bereich der Abwinklung 15 der Arme 3, 4 zwischen den Abschnitten 6 und 8 bzw. 7 und 9 verformt, so daß die Arme 3, 4 danach das Hohlorgan 12 fest zwischen sich einschließend aneinander anlegen und im wesentlichen gestreckt sind, wie dies in Figur 5 dargestellt ist. Die Arme 3, 4 weisen dabei zwei nebeneinander liegende Stege auf, sie sind also als Doppelclip ausgebildet und liegen in zwei nebeneinander liegenden Bereichen am Hohlorgan an, das auf diese Weise sicher von dem Ligaturclip 1 gefaßt wird.

Bei dem Ausführungsbeispiel der Figur 2 sind die Klemmbacken 13, 14 des Anlegeinstrumentes 11 geringfügig breiter ausgebildet als der Ligaturclip 1, zur Zentrierung des Ligaturclips 1 tragen die Klemmbacken 13, 14 an ihren Außenkanten seitlich nach oben überstehende Leisten 28, 29, die an den Außenseiten der Abschnitte 6, 7, 8, 9 anliegen und damit eine sichere Positionierung des Ligaturclips zwischen den Klemmbacken sicherstellen.

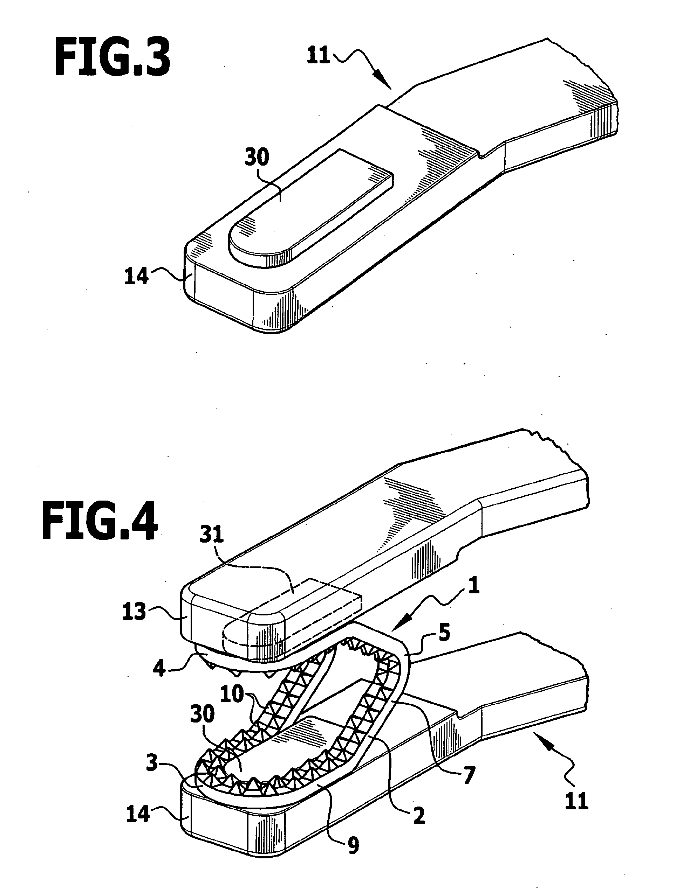

In den Figuren 3 und 4 ist ein weiteres Ausführungsbeispiel eines derartigen Anlegeinstrumentes 11 dargestellt, bei dem die Klemmbacken 13, 14 Zentriervorsprünge 30, 31 tragen, die genau in den Zwischenraum zwischen den Abschnitten 6, 7 bzw. 8, 9 der Arme 3, 4 hineinpassen, wenn die Klemmbacken 13, 14 an die Außenseiten der Arme 3, 4 angelegt sind. Auch auf diese Weise ergibt sich eine exakte und dauerhafte Positionierung des Ligaturclips zwischen den Klemmbacken 13, 14, bei diesem Ausführungsbeispiel können die Klemmbacken 13, 14 gegebenenfalls auch schmaler ausgebildet sein als die Ligaturclips 1.

In Figur 15 ist schematisch ein im Querschnitt rechteckiger Träger 16 dargestellt, auf den eine größere Anzahl von Ligaturclips 1 der in den Figuren 1 bis 3 dargestellten Art in Offenstellung aufgeschoben sind. Die Abmessungen des Trägers 16 sind dabei so gewählt, daß seine Breite der Breite des Zwischenraumes zwischen den nebeneinander verlaufenden Stegen der Arme 3, 4 entspricht, so daß die Abschnitte 6, 7, 8, 9 der Arme 3, 4 dicht am Träger 16 anliegen und die Ligaturclips 1 daher kraftschlüssig auf dem Träger 16 festlegen. Alle Ligaturclips 1 sind dabei parallel zueinander und unmittelbar nebeneinander angeordnet, so daß auf engem Raum eine größere Anzahl derartiger Ligaturclips 1 auf dem Träger 16 gelagert werden können. Mittels eines in der Zeichnung nur schematisch dargestellten Ausschubgliedes 17 können die Ligaturclips 1 auf dem Träger 16 verschoben werden, so daß auf diese Weise jeweils der vorderste Ligaturclip 1 für die Anlage freigegeben werden kann. Eine solche Freigabe kann auch durch andere Mittel erfolgen, beispielsweise durch ein Anlegeinstrument 11, welches aus dem Vorrat von Ligaturclips 1 auf dem Träger 16 jeweils nur den vordersten Ligaturclip oder nur den hintersten Ligaturclip erfaßt und vom Träger abziehen kann. Wesentlich ist, daß durch die Ausgestaltung der Ligaturclips 1 mit zwei nebeneinander liegenden Stegen zwischen den Stegen ein Aufnahmeraum für den Träger gebildet wird, der ohne weitere Hilfsmittel die Lagerung der Ligaturclips 1 auf einem in diesen Aufnahmeraum eingeschobenen Träger ermöglicht.

In den Figuren 16 bis 18 ist ein abgewandeltes Ausführungsbeispiel eines Ligaturclips 1 beschrieben. Dieser ist ähnlich aufgebaut wie der Ligaturclip der vorstehend beschriebenen Ausführungsbeispiele, einander entsprechende Teile tragen daher dieselben Bezugszeichen.

Bei dem Ligaturclip der Figuren 16 bis 18 gehen die nebeneinander liegenden Abschnitte 8 und 9 am freien Ende der Arme 3, 4 nicht in Form eines Bogens unmittelbar ineinander über, sondern an die freien Enden der Abschnitte 8 und 9 schließen sich nach außen und damit nach gegenüberliegenden Seiten abstehende Transversalabschnitte 32, 33 an, die in derselben Ebene verlaufen wie die nebeneinander liegenden Abschnitte 8 und 9. An ihren den Abschnitten 8 und 9 abgewandten äußeren Enden 34, 35 gehen die Transversalabschnitte 32, 33 über kreisbogenförmige, sich etwa über 180 ° erstreckende Endabschnitte 36, 37 über in einen gemeinsamen Klemmabschnitt 38, der auf der den nebeneinander liegenden Abschnitten 8, 9 abgewandten Seite der Transversalabschnitte 32, 33 im Abstand zu diesen verläuft und die beiden Endabschnitte 36, 37 miteinander verbindet. Der Klemmabschnitt 38 verläuft dabei bei dem in den Figuren 16 bis 18 dargestellten Ausführungsbeispiel zwischen den Endabschnitten 36 und 37 kreisbogenförmig, wobei die äußeren Enden 34, 35 nach außen gebogen sind, das heißt also, von den Armen 3, 4 wegweisend. Der Klemmabschnitt 38 liegt in derselben Ebene wie die Transversalabschnitte 32, 33 und die bogenförmigen Endabschnitte 36, 37, die Transversalabschnitte 32, 33, die Endabschnitte 36, 37 und der Klemmabschnitt 38 bilden eine gemeinsame Klemmfläche am freien Ende der Arme 3, 4 aus.

Bei dem Ausführungsbeispiel der Figur 16 stehen die Transversalabschnitte 32, 33 unter einem Winkel von etwa 45° von den nebeneinander liegenden Abschnitten 8, 9 ab, es kann sich dabei aber auch um einen Winkel in der Größenordnung von 90° handeln. Insbesondere können die Transversalabschnitte 32, 33 und der Klemmabschnitt 38 parallel zueinander verlaufen. In jedem Fall verbleibt zwischen den Transversalabschnitten 32, 33 und dem Klemmabschnitt 38 ein Zwischenraum 39.

Auch der Ligaturclip der Figuren 16 bis 18 ist einstückig aus einem Band 2 geformt und trägt auf seiner Innenseite Zähne 10 oder eine ähnliche Profilierung.

Außerdem sind an einem der beiden Klemmabschnitte 38 über dessen Länge verteilt mehrere stiftförmige Vorsprünge 40 angeordnet, denen jeweils im anderen Klemmabschnitt eine Bohrung 41 gegenübersteht, in die der stiftförmige Vorsprung 40 eintaucht, wenn die beiden Klemmabschnitte 38 der beiden Arme 3, 4 einander angenähert werden.

Der in den Figuren 16 bis 18 dargestellte Ligaturclip ist besonders geeignet zur Verbindung von zwei Hohlorganen 12. Diese werden an den aneinanderstossenden Enden mit ihrem Rand 42 seitlich nach außen umgebogen, so daß ein flanschförmiger Überstand entsteht. In diesem Bereich können mehrere Ligaturclips in Umfangsrichtung nebeneinander derart angeordnet werden, daß die bogenförmigen Klemmabschnitte 38 der Außenkontur der Hohlorgane 12 folgen.

Es ist dabei insbesondere günstig, wenn Ligaturclips verwendet werden, bei denen die Krümmung der Klemmabschnitte 38 der Krümmung der Hohlorgane entspricht. Zu diesem Zweck werden in einem Satz dem Operateur Ligaturclips mit Klemmabschnitten 38 zur Verfügung gestellt, die eine unterschiedliche Krümmung aufweisen, so daß er aus diesem Satz die Ligaturclips auswählen kann, die jeweils an die Krümmung des Hohlorgans angepaßt sind.

Wenn die Ligaturclips angelegt und so geschlossen sind, daß die Klemmabschnitte 38 der beiden Arme 3, 4 gegeneinander gedrückt sind, dann nehmen die Klemmabschnitte 38 und gegebenenfalls auch die Transversalabschnitte 32, 33 zwischen sich das Gewebe auf, im Beispiel der Verbindung der Hohlorgane 12 also den Rand 42, wie dies in Figur 18 dargestellt ist. Die stiftförmigen Vorsprünge 40 durchdringen dabei den Rand 42 und treten in die Bohrung 41 des gegenüberliegenden Klemmabschnittes 38 ein, so daß eine sichere Fixierung der Ligaturclips am Rand 42 sichergestellt ist. Diese sichere Fixierung wird auch dadurch unterstützt, daß das Gewebe des Randes 42 in den Zwischenraum 39 zwischen dem Klemmabschnitt 38 einerseits und den beiden Transversalabschnitten 32, 33 eintritt und dadurch eine seitliche Verschiebung des Ligaturclips gegenüber dem Rand verhindert.

## Patentansprüche

1. Chirurgischer Ligaturclip (1) mit zwei Haltearmen (3, 4), die an jeweils einem Ende über eine verformbare Verbindungsstelle (5) miteinander verbunden sind und derart gegeneinander biegbar sind, daß die Arme (3, 4) aus einer Offenstellung, in der sie einen größeren Abstand voneinander haben, in eine Schließstellung gelangen, in der die einander zugewandten Innenseiten der Arme (3, 4) dauerhaft einander angenähert sind, wobei der Ligaturclip (1) aus einem in sich geschlossenen Steg (2) besteht, der im Bereich der beiden Arme (3, 4) und der Verbindungsstelle (5) zwei nebeneinander liegende Abschnitte (6, 8; 7, 9) ausbildet, die an den der Verbindungsstelle (5) abgewandten freien Enden der Arme (3, 4) ineinander übergehen, **dadurch gekennzeichnet, dass** die nebeneinander liegenden Abschnitte (8, 9) an ihrem der Verbindungsstelle (5) abgewandten Ende in nach gegenüberliegenden Seiten seitlich abstehende Transversalabschnitte (32, 33) einmünden.

2. Ligaturclip nach Anspruch 1, **dadurch gekennzeichnet, daß** der Steg (2) über seine gesamte Länge eine gleichbleibende Breite aufweist.

3. Ligaturclip nach Anspruch 1 oder 2, **dadurch gekennzeichnet, daß** der Steg (2) über seine gesamte Länge eine gleichbleibende Höhe aufweist.

4. Ligaturclip nach einem der voranstehenden Ansprüche, **dadurch gekennzeichnet, daß** der Steg (2) über seine gesamte Länge einen gleichbleibenden Querschnitt aufweist.

5. Ligaturclip nach einem der voranstehenden Ansprüche, **dadurch gekennzeichnet, daß** der Steg (2) über seine gesamte Länge einen rechteckigen Querschnitt aufweist.

6. Ligaturclip nach einem der voranstehenden Ansprüche, **dadurch gekennzeichnet, daß** die nebeneinander liegenden Abschnitte (6, 8; 7, 9) geradlinig ausgebildet sind.

7. Ligaturclip nach Anspruch 6, **dadurch gekennzeichnet, daß** die nebeneinander liegenden Abschnitte (6, 8; 7, 9) parallel zueinander verlaufen.

8. Ligaturclip nach einem der voranstehenden Ansprüche, **dadurch gekennzeichnet, daß** die Transversalabschnitte (32, 33) an ihren den nebeneinander liegenden Abschnitten (8, 9) abgewandten äußeren Enden (34, 35) in einen Klemmabschnitt (38) übergehen, der im Abstand von den Transversalabschnitten (32, 33) auf deren den nebeneinander liegenden Abschnitten (8, 9) abgewandter Seite verläuft.

9. Ligaturclip nach Anspruch 8, **dadurch gekennzeichnet, daß** der Klemmabschnitt (38) im wesentlichen parallel zu den Transversalabschnitten (32, 33) verläuft.

10. Ligaturclip nach Anspruch 8 oder 9, **dadurch gekennzeichnet, daß** die Transversalabschnitte (32, 33) an den äußeren Enden (34, 35) bogenförmig in den Klemmabschnitt (38) übergehen.

11. Ligaturclip nach Anspruch 10, **dadurch gekennzeichnet, daß** die Transversalabschnitte (32, 33) an den äußeren Enden (34, 35) kreisbogenförmig in den Klemmabschnitt (38) übergehen.

12. Ligaturclip nach einem der voranstehenden Ansprüche, **dadurch gekennzeichnet, daß** die Transversalabschnitte (32, 33) und gegebenenfalls der Klemmabschnitt (38) in derselben Ebene liegen wie die an die Transversalabschnitte (32, 33) anschließenden nebeneinander liegenden Abschnitte (8, 9).

13. Ligaturclip nach einem der Ansprüche 8 bis 12, **dadurch gekennzeichnet, daß** der Klemmabschnitt (38) bogenförmig verläuft.

14. Ligaturclip nach Anspruch 13, **dadurch gekennzeichnet, daß** der Klemmabschnitt (38) kreisbogenförmig verläuft.

15. Ligaturclip nach einem der Ansprüche 13 oder 14, **dadurch gekennzeichnet, daß** der Klemmabschnitt (38) von den nebeneinander liegenden Abschnitten (8, 9) wegweisend gebogen ist.

16. Ligaturclip nach einem der Ansprüche 8 bis 15, **dadurch gekennzeichnet, daß** mindestens ein Klemmabschnitt (38) einen Vorsprung (40) trägt, der bei aneinander liegenden Klemmabschnitten (38) der beiden Arme (3, 4) in eine Ausnehmung (41) des anderen Klemmabschnitt (38) eintaucht.

17. Ligaturclip nach Anspruch 16, **dadurch gekennzeichnet, daß** der Vorsprung (40) ein Stift ist.

18. Ligaturclip nach Anspruch 16 oder 17, **dadurch gekennzeichnet, daß** an dem Klemmabschnitt (38) eines Armes (3, 4) über dessen Längsrichtung verteilt mehrere Vorsprünge (40) und an dem Klemmabschnitt (38) des anderen Armes entsprechende Ausnehmungen (40) angeordnet sind.

19. Ligaturclip nach einem der voranstehenden Ansprüche, **dadurch gekennzeichnet, daß** der Steg (2) an seiner die Innenseite der Arme (3, 4) ausbildenden Seite profiliert ist.

20. Ligaturclip nach Anspruch 19, **dadurch gekennzeichnet, daß** die Profilierung im Bereich des freien Endes der Arme (3, 4) eine Mauszahnprofilierung ist.

21. Ligaturclip nach einem der voranstehenden Ansprüche, **dadurch gekennzeichnet, daß** die Arme (3, 4) an ihren freien Enden eine Rastvorrichtung tragen, die beim Annähern der Arme in diesem Bereich zu einer Rastverbindung der Arme führt.

22. Ligaturclip nach Anspruch 21, **dadurch gekennzeichnet, daß** die Rastvorrichtung mindestens einen Vorsprung (22; 26) an einem Arm (4) umfaßt, der bei geschlossenem Ligaturclip (1) einen Teil des anderen Armes (3) hintergreift.

23. Ligaturclip nach Anspruch 22, **dadurch gekennzeichnet, daß** der Vorsprung (22) an seinem freien Ende eine Verbreiterung (24) oder eine Abwinkelung (23) aufweist.

24. Ligaturclip nach Anspruch 22, **dadurch gekennzeichnet, daß** der Vorsprung (26) als Haken ausgebildet ist.

25. Ligaturclip nach einem der voranstehenden Ansprüche, **dadurch gekennzeichnet, daß** der Steg (2) ein einteiliges Bauteil ist.

26. Ligaturclip nach einem der voranstehenden Ansprüche, **dadurch gekennzeichnet, daß** die beiden nebeneinanderliegenden Abschnitte (6, 7; 8, 9) im Bereich der Verbindungsstelle (5) durch einen Steg (27) miteinander verbunden sind.

## Claims

1. Surgical ligature clip (1) comprising two retaining arms (3, 4), which are connected to one another in each case at one end by a deformable connection point (5) and bendable in such a way towards one another that the arms (3, 4) move from an open position, in which they are spaced further apart from one another, into a closed position, in which the mutually opposing inner sides of the arms (3, 4) are brought permanently closer to one another, the ligature clip (1) consisting of an endless web member (2), which in the region of the two arms (3, 4) and the connection point (5) forms two juxtaposed portions (6, 8; 7, 9) that merge into one other at the free ends of the arms (3, 4) remote from the connection point (5), **characterized in that** the juxtaposed portions (8, 9) at their end remote from the connection point (5) open into transverse portions (32, 33) that project laterally in opposite directions.

2. Ligature clip according to claim 1, **characterized in that** the web member (2) has a constant width over its entire length.

3. Ligature clip according to claim 1 or 2, **characterized in that** the web member (2) has a constant height over its entire length.

4. Ligature clip according to one of the preceding claims, **characterized in that** the web member (2) has a constant cross section over its entire length.

5. Ligature clip according to one of the preceding claims, **characterized in that** the web member (2) has a rectangular cross section over its entire length.

6. Ligature clip according to one of the preceding claims, **characterized in that** the juxtaposed portions (6, 8; 7, 9) are of a rectilinear form.

7. Ligature clip according to claim 6, **characterized in that** the juxtaposed portions (6, 8; 7, 9) extend parallel to one another.

8. Ligature clip according to one of the preceding claims, **characterized in that** the transverse portions (32, 33) at their outer ends (34, 35) remote from the juxtaposed portions (8, 9) merge into a clamping portion (38) that extends at a spacing from the transverse portions (32, 33) at the side thereof remote from the juxtaposed portions (8, 9).

9. Ligature clip according to claim 8, **characterized in that** the clamping portion (38) extends substantially parallel to the transverse portions (32, 33).

10. Ligature clip according to claim 8 or 9, **characterized in that** the transverse portions (32, 33) at the outer ends (34, 35) merge in an arcuate manner into the clamping portion (38).

11. Ligature clip according to claim 10, **characterized in that** the transverse portions (32, 33) at the outer ends (34, 35) merge in a circular-arcuate manner into the clamping portion (38).

12. Ligature clip according to one of the preceding claims, **characterized in that** the transverse portions (32, 33) and optionally the clamping portion (38) lie in the same plane as the juxtaposed portions (8, 9) adjoining the transverse portions (32, 33).

13. Ligature clip according to one of claims 8 to 12, **characterized in that** the clamping portion (38) extends in an arcuate manner.

14. Ligature clip according to claim 13, **characterized in that** the clamping portion (38) extends in a circular arcuate manner.

15. Ligature clip according to one of claims 13 or 14, **characterized in that** the clamping portion (38) is bent in a direction away from the juxtaposed portions (8, 9).

16. Ligature clip according to one of claims 8 to 15, **characterized in that** at least one clamping portion (38) carries a projection (40) that, when the clamping portions (38) of the two arms (3, 4) lie adjacent to one another, engages into a recess (41) of the other clamping portion (38).

17. Ligature clip according to claim 16, **characterized in that** the projection (40) is a pin.

18. Ligature clip according to claim 16 or 17, **characterized in that** a plurality of projections (40) are disposed on, and distributed over the longitudinal direction of, the clamping portion (38) of one arm (3, 4) and corresponding recesses (40) are disposed on the clamping portion (38) of the other arm.

19. Ligature clip according to one of the preceding claims, **characterized in that** the web member (2) is profiled on its side forming the inner side of the arms (3, 4).

20. Ligature clip according to claim 19, **characterized in that** the profiling in the region of the free end of the arms (3, 4) is mouse-tooth profiling.

21. Ligature clip according to one of the preceding claims, **characterized in that** the arms (3, 4) on their free ends carry a detent device that, when the arms are brought closer to one another, leads to a detent connection of the arms in this region.

22. Ligature clip according to claim 21, **characterized in that** the detent device comprises at least one projection (22; 26) on one arm (4) that in the closed state of the ligature clip (1) engages behind a part of the other arm (3).

23. Ligature clip according to claim 22, **characterized in that** the projection (22) has on its free end a widened portion (24) or a bent portion (23).

24. Ligature clip according to claim 22, **characterized in that** the projection (26) takes the form of a hook.

25. Ligature clip according to one of the preceding claims, **characterized in that** the web member (2) is an integral component.

26. Ligature clip according to one of the preceding claims, **characterized in that** the two juxtaposed portions (6, 7; 8, 9) are connected to one another in the region of the connection point (5) by means of a web (27).

## Revendications

1. Pince de ligature chirurgicale (1) comprenant deux bras de maintien (3, 4) qui sont reliés l'un à l'autre à une extrémité respective via un emplacement de liaison déformable (5), et qui peuvent être cintrés l'un vers l'autre de telle façon que les bras (3, 4), en partant d'une position ouverte dans laquelle ils présentent une grande distance l'un par rapport à l'autre, parviennent dans une position fermée dans laquelle les faces intérieures, tournées l'une vers l'autre, des bras (3, 4) sont rapprochées durablement l'une de l'autre, ladite pince de ligature (1) étant formée d'une barrette (2) refermée sur elle-même qui, dans la région des deux bras (3, 4) et de l'emplacement de liaison (5), forme deux portions (6, 8 ; 7, 9) situées l'une à côté de l'autre, qui se transforment l'une dans l'autre au niveau des extrémités libres, détournées de l'emplacement de liaison (5), des bras (3, 4),
**caractérisée en ce que** les portions (8, 9) situées l'une à côté de l'autre débouchent, au niveau de leur extrémité détournée de l'emplacement de liaison (5), dans des portions transversales (32, 33) qui dépassent latéralement vers des côtés opposés.

2. Pince de ligature selon la revendication 1, **caractérisée en ce que** la barrette (2) présente une largeur constante sur toute sa longueur.

3. Pince de ligature selon la revendication 1 ou 2, **caractérisée en ce que** la barrette (2) présente une hauteur constante sur toute sa longueur.

4. Pince de ligature selon l'une des revendications précédentes, **caractérisée en ce que** la barrette (2) présente une section constante sur toute sa longueur.

5. Pince de ligature selon l'une des revendications précédentes, **caractérisée en ce que** la barrette (2) présente une section rectangulaire sur toute sa longueur.

6. Pince de ligature selon l'une des revendications précédentes, **caractérisée en ce que** les portions situées l'une à côté de l'autre (6, 8 ; 7, 9) sont réalisées rectilignes.

7. Pince de ligature selon la revendication 6, **caractérisée en ce que** les portions situées l'une à côté de l'autre (6, 8 ; 7, 9) s'étendent parallèlement l'une à l'autre.

8. Pince de ligature selon l'une des revendications précédentes, **caractérisée en ce que** les portions transversales (32, 33) se transforment, à leurs extrémités extérieures (34, 35) détournées des portions (8, 9) situées l'une à côté de l'autre, en une portion de serrage (38) qui s'étend à distance des portions transversales (32, 33) sur leur côté détourné des portions (8, 9) situées l'une à côté de l'autre.

9. Pince de ligature selon la revendication 8, **caractérisée en ce que** la portion de serrage (38) s'étend essentiellement parallèlement aux portions transversales (32, 33).

10. Pince de ligature selon la revendication 8 ou 9, **caractérisée en ce que** les portions transversales (32, 33) se transforment dans la portion de serrage (38) à la manière d'un arc à leurs extrémités extérieures (34, 35).

11. Pince de ligature selon la revendication 10, **caractérisée en ce que** le les portions transversales (32, 33) se transforment dans la portion de serrage (38) à la manière d'un arc circulaire à leurs extrémités extérieures (34, 35).

12. Pince de ligature selon l'une des revendications précédentes, **caractérisée en ce que** les portions transversales (32, 33) et le cas échéant la portion de serrage (38) sont situées dans le même plan que les portions (8, 9) situées l'une à côté de l'autre qui font suite aux portions transversales (32, 33).

13. Pince de ligature selon l'une des revendications 8 à 12, **caractérisée en ce que** la portion de serrage (38) s'étend à la manière d'un arc.

14. Pince de ligature selon la revendication 13, **caractérisée en ce que** la portion de serrage (38) s'étend à la manière d'un arc circulaire.

15. Pince de ligature selon l'une des revendications 13 ou 14, **caractérisée en ce que** la portion de serrage (38) est cintrée en s'éloignant des portions situées l'une à côté de l'autre (8, 9).

16. Pince de ligature selon l'une des revendications 8 à 15, **caractérisée en ce qu'**au moins une portion de serrage (38) porte une saillie (40) qui, lorsque les portions de serrage (38) des deux bras (3, 4) sont situées l'une contre l'autre, plonge dans un évidement (41) de l'autre portion de serrage (38).

17. Pince de ligature selon la revendication 16, **caractérisée en ce que** la saillie (40) est une tige.

18. Pince de ligature selon la revendication 16 ou 17, **caractérisée en ce que** plusieurs saillies (40) sont agencées sur la portion de serrage (38) d'un bras (3, 4) de façon répartie sur sa direction longitudinale, et des évidements correspondants (40) sont agencés sur la portion de serrage (38) de l'autre bras.

19. Pince de ligature selon l'une des revendications précédentes, **caractérisée en ce que** la barrette (2) est profilée sur son côté qui forme la face intérieure des bras (3, 4).

20. Pince de ligature selon la revendication 19, **caractérisée en ce que** le profilage dans la région de l'extrémité libre des bras (3, 4) est un profilage en forme de "dents de souris".

21. Pince de ligature selon l'une des revendications précédentes, **caractérisée en ce que** les bras (3, 4) portent à leurs extrémités libres un dispositif d'enclenchement qui, lors du rapprochement des bras, mène à une liaison des bras par enclenchement dans cette région.

22. Pince de ligature selon la revendication 21, **caractérisée en ce que** le dispositif d'enclenchement comprend au moins une saillie (22 ; 26) sur un bras (4) qui, lorsque la pince de ligature (1) est fermée, engage une partie de l'autre bras (3) par l'arrière.

23. Pince de ligature selon la revendication 22, **caractérisée en ce que** la saillie (22) comporte à son extrémité libre un élargissement (24) ou un coudage (23).

24. Pince de ligature selon la revendication 22, **caractérisée en ce que** la saillie (26) est réalisée sous forme de crochet.

25. Pince de ligature selon l'une des revendications précédentes, **caractérisée en ce que** la barrette (2) est un composant d'un seul tenant.

26. Pince de ligature selon l'une des revendications précédentes, **caractérisée en ce que** les deux portions (6, 7 ; 8, 9) situées l'une à côté de l'autre sont reliées l'une à l'autre par une barrette (27) dans la région de l'emplacement de liaison (5).
